# EUROPÄISCHE PATENTSCHRIFT

(11) **EP 3 277 222 B1**
(45) Veröffentlichungstag und Bekanntmachung des Hinweises auf die Patenterteilung: **07.07.2021**
(21) Anmeldenummer: 16721351.1
(22) Anmeldetag: 23.03.2016
(51) Int. Cl.: A61F 2/24

(54) **HERZKLAPPENPROTHESE**
HEART VALVE PROSTHESIS
PROTHÈSE VALVULAIRE CARDIAQUE

(30) Priorität: 02.04.2015 DE 102015206097
(43) Veröffentlichungstag der Anmeldung: 07.02.2018
(73) Patentinhaber: Sievers, Hans-Hinrich, 24119 Kronshagen (DE)
(72) Erfinder: SIEVERS, Hans-Hinrich, 24119 Kronshagen (DE); SCHARFSCHWERDT, Michael, 23564 Lübeck (DE); HOF, Andreas, 23568 Lübeck (DE)
(74) Vertreter: Patentanwälte Vollmann Hemmer Lindfeld Partnerschaft mbB
(86) Internationale Anmeldenummer: PCT/DE2016/200152
(87) Internationale Veröffentlichungsnummer: WO 2016/155731

(56) Entgegenhaltungen:
- US-A1- 2004 176 839
- US-A1- 2007 005 134
- US-A1- 2012 059 458
- US-A1- 2014 039 613
- US-B1- 6 951 573

## Beschreibung

Die Erfindung betrifft eine Herzklappenprothese mit einem Nahtring zum Vernähen in einem Blutgefäß.

Es sind Herzklappenprothesen, insbesondere biologische Herzklappenprothesen bekannt, welche einen bogenförmig verlaufenden Nahtring (scalloped Nahtring) aufweisen, entlang dem die Herzklappenprothese mit einem Blutgefäß vernäht werden kann. Problematisch dabei ist, dass der bogenförmige Verlauf des Nahtringes nicht für alle Anatomien passend ist, so dass das Vernähen in Einzelfällen nur schwer oder mit einer unerwünschten Verformung des Blutgefäßes oder der Herzklappenprothese möglich ist.

US 2004/0176839 A1 offenbart eine Herzklappenprothese mit einem beweglichen Nahtring. Dabei bildet der Nahtring einen geschlossenen Ring, welcher die Herzklappenprothese umfänglich umgibt. Mit diesem geschlossenen Ring ist eine Anpassung an verschiedene anatomische Verhältnisse nur eingeschränkt möglich.

US 2012/0059458 A1 offenbart eine Herzklappentragstruktur, welche zum Einsetzen mit Hilfe eines Katheters ausgebildet ist und welche nicht dem Vernähen mit dem Gewebe dient.

Im Hinblick auf diese Problematik ist es Aufgabe der Erfindung, eine Herzklappenprothese derart zu verbessern, dass sie bei einer Vielzahl unterschiedlicher Anatomien und insbesondere bei asymmetrisch geformten Aortenklappen problemlos in ein Blutgefäß einnähbar ist.

Diese Aufgabe wird durch eine Herzklappenprothese mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Die erfindungsgemäße Herzklappenprothese ist insbesondere eine Herzklappenprothese zum Ersatz der Aortenklappe. Die erfindungsgemäße Herzklappenprothese weist in der vorgesehenen Strömungsrichtung des Blutes ein erstes stromabwertiges Axialende und ein zweites stromaufwärts gelegenes Axialende auf. Ferner weist die Herzklappenprothese einen Nahtring auf, welcher dazu dient, die Herzklappenprothese in ein Blutgefäß einzunähen. Dieser Nahtring ist vorzugsweise im Bereich des zweiten Axialendes gelegen oder näher zu dem zweiten Axialende als zu dem ersten Axialende gelegen. Der Nahtring hat einen bogenförmigen Verlauf, welcher sich aus drei Bögen zusammensetzt, welche mit ihren Enden aneinanderstoßen bzw. ineinander übergehen. Dort, wo die Bögen aneinanderstoßen bzw. ineinander übergehen, werden Übergangsbereiche gebildet, welche eine im Wesentlichen V-förmige Form aufweisen, wobei diese V-förmige Form zu dem zweiten Axialende hin geöffnet ist, während die Bögen zum ersten Axialende hin geöffnet sind.

An zumindest einem der so gebildeten Übergangsbereiche weist der Nahtring ferner eine bewegliche Lasche auf. Diese bewegliche Lasche hat eine größere freie Länge als die übrigen Bereiche des Nahtringes. Die Lasche ist so ausgebildet, dass sie wahlweise zu dem ersten Axialende hin gerichtet sein kann oder zu dem zweiten Axialende hin umgeklappt werden kann. Das bedeutet, die Lasche weist eine mit dem Nahtring verbundene Basis bzw. Basiskante auf, von welcher sie sich zu einem freien Ende hin wegerstreckt, wobei dieses freie Ende entweder zu dem ersten Axialende hin gerichtet oder zu dem zweiten Axialende hin gerichtet sein kann, je nachdem in welche Richtung die Lasche geklappt wird. Die Umklappbarkeit der Lasche ermöglicht es, den von der Lasche gebildeten Teil des Nahtringes so zu richten bzw. zu positionieren, wie es die Anatomie des Patienten erfordert. So wird eine größere Flexibilität des Vernähens ermöglicht, da die Naht nicht zwingend dem bogenförmigen Verlauf des Nahtringes folgen muss, sondern dort, wo die Lasche angeordnet ist, durch entsprechendes Klappen bzw. Ausrichten der Lasche die erforderlichen Nähte auch an anderer Position gesetzt werden können.

Vorzugsweise ist an zweien oder jedem der drei Übergangsbereiche eine derartige bewegliche Lasche angeordnet bzw. ausgebildet, welche wahlweise zu dem ersten Axialende gerichtet oder zu dem zweiten Axialende hin umgeklappt werden kann. So besteht bevorzugt in jedem der Übergangsbereiche die Flexibilität, die Naht entweder dem bogenförmigen Verlauf des Nahtringes folgen zu lassen oder aber auch von diesem Verlauf abweichend die Herzklappenprothese mit dem Blutgefäß vernähen zu können, wenn die Lasche entsprechend gerichtet bzw. umgeklappt wird.

Vorzugsweise ist der Nahtring so ausgestaltet, dass, wenn die Lasche oder die Laschen zu dem ersten Axialende hin gerichtet sind, der Nahtring streifenförmig ausgebildet ist und entlang seines bogenförmigen Verlaufs eine im Wesentlichen konstante Breite aufweist. Das heißt, der Nahtring ist so ausgebildet, dass die Lasche, wenn sie zu dem ersten Axialende hin gerichtet bzw. geklappt ist, eine Form definiert, welche dem bekannten bogenförmigen Verlauf des Nahtringes entspricht. Vorzugsweise kommt die Lasche dabei auf darunterliegenden Teilen des Nahtringes zum Liegen bzw. überdeckt einen radial weiter innenliegenden Teil des Nahtringes. Die Lasche definiert, wenn sie zu dem ersten Axialende hin gerichtet ist, vorzugsweise die Spitze des Übergangsbereiches zwischen zwei Bögen des Nahtringes. So kann ein Nahtverlauf in der bekannten durch den bogenförmigen Nahtring definierten Kontur realisiert werden.

An seiner dem zweiten Axialende zugewandten Seite bzw. Seitenkante weist der Nahring vorzugsweise eine bogenförmige aus drei Bögen gebildete Kontur auf. Das heißt zum zweiten Nahtende hin definiert der Nahtring, wenn die Lasche zu dem ersten Axialende hin geklappt ist, die beschriebene aus drei Bögen zusammengesetzte Bogenform, wobei unterhalb der Übergangsbereiche, das heißt zwischen den Übergangsbereichen und dem zweiten Axialende, zwischen den aneinander angrenzenden Bögen Freiräume bzw. freie, nicht vom Nahtring überdeckte Zwickel gebildet werden. So wird eine Bogenform des Nahtringes erreicht, welche dem bogenförmigen Verlauf der Ränder einer normal ausgebildeten natürlichen Aortenklappe entspricht und sich so in die übliche Anatomie des Blutgefäßes im Bereich der Aortenklappe optimal einfügen lässt.

Weiter bevorzugt ist die Lasche bzw. sind die Laschen jeweils so ausgebildet, dass sie, wenn sie zu dem zweiten Axialende hin gerichtet bzw. umgeklappt sind, eine zwischen zwei aneinander angrenzenden Bögen gebildete Einbuchtung des Nahtringes zumindest teilweise überdecken. Diese Einbuchtung ist der Freiraum, welcher sich durch die zu dem zweiten Axialende geöffnete V-förmige Form des Übergangsbereiches gebildet wird. Dies ist der oben genannte freie Zwickel zwischen den benachbarten Bögen. Dadurch, dass dieser durch die Lasche zumindest teilweise überdeckt werden kann, kann auch in diesem Bereich wahlweise zu dem bogenförmigen Verlauf ein Vernähen ermöglicht werden, um eine Anpassung an verschiedene Anatomien, insbesondere an asymmetrische Anatomien, zu ermöglichen.

Besonders bevorzugt ist die Lasche bzw. sind die Laschen derart geformt, dass sie, wenn sie zu dem zweiten Axialende hin umgeklappt sind, sich in axialer Richtung zu diesem zweiten Axialende hin zumindest bis zu einer Umfangslinie erstrecken, welche durch die dem zweiten Axialende zugewandten Scheitelpunkte der Bögen des Nahtringes verläuft. Das heißt die Spitzen der Laschen erstrecken sich, wenn sie zu dem zweiten Axialende hin gerichtet bzw. umgeklappt sind, in axialer Richtung zu dem zweiten Axialende hin im Wesentlichen genau so weit, wie die Bögen. Dies ermöglicht einen im Wesentlichen ringförmigen Nahtverlauf, wenn die Laschen in dieser Weise ausgerichtet sind.

Besonders bevorzugt ist der Nahtring somit derart ausgebildet, dass, wenn die Laschen zu dem zweiten Axialende hin gerichtet bzw. umgeklappt sind, ein im Wesentlichen kreisringförmiger oder kreisabschnittförmiger Nahtbereich definiert wird, entlang welchem die Herzklappenprothese mit einem Blutgefäß vernäht werden kann. Der kreisringförmige Nahtbereich erstreckt sich dabei über die Lasche bzw. Laschen und über die an die Scheitelpunkte angrenzenden Abschnitte der Bögen. Es ist zu verstehen, dass je nach Anatomie, alle drei Laschen, sofern drei Laschen vorgesehen sind, in dieser Weise ausgerichtet werden können. Alternativ ist es jedoch auch möglich, beispielsweise lediglich eine Lasche zu dem zweiten Axialende hin umzuklappen und zwei andere Laschen in den beiden anderen Übergangsbereichen in ihrer zum ersten Axialende hin gerichteten Lage zu belassen.

Weiter bevorzugt ist der Nahtring derart ausgestaltet, dass, wenn die Laschen zu dem ersten Axialende hin gerichtet sind, der Nahtring einen bogenförmigen Nahtbereich definiert, welcher derart verläuft, dass er ein Vernähen in der Aorta entlang des normalen Verlaufes des Randes der natürlichen Klappensegel der Aortenklappe ermöglicht. So kann die Herzklappenprothese mit einem Nahtverlauf vernäht werden, welche der üblichen Anatomie der natürlichen Aortenklappen folgt. Bei einer von dieser üblichen Anatomie abweichenden Anatomie, beispielsweise, wenn lediglich zwei natürliche Klappensegel vorhanden waren, kann gegebenenfalls zumindest eine Lasche in der oben beschriebenen Weise zu dem zweiten Axialende hin umgeklappt werden. Daher weist der Nahtring vorzugsweise zumindest an einem Übergangsbereich eine Lasche, wie sie vorangehend beschrieben wurde, auf.

Weiter bevorzugt ist der Nahtring derart ausgebildet, dass die Laschen, wenn sie zu dem ersten Axialende hin gerichtet sind, die dem ersten Axialende zugewandten axialen Endbereiche des Nahtringes definieren. Wenn lediglich eine Lasche vorgesehen ist, wird nur in demjenigen Übergangsbereich, an welchem die Lasche angeordnet ist, der axiale Endbereich des Nahtringes durch die Lasche definiert. In den beiden anderen Übergangsbereichen ist dann ein durchgehender Verlauf des Nahtringes ohne derartige Laschen vorgesehen. Wenn in allen Übergangsbereichen derartige Laschen vorgesehen sind, definieren die Laschen in allen Übergangsbereichen den dem ersten Axialende zugewandten axialen Endbereich des Nahtringes. Wenn die Lasche bzw. die Laschen zum zweiten Axialende hin umgeklappt sind, wird der dem ersten Axialende zugewandte Endbereich des Nahtringes entweder durch die Seitenkante der Lasche, um welche die Lasche geklappt wird, definiert oder einen unterhalb der Lasche, das heißt einen radial weiter innenliegenden Bereich des Nahtringes, definiert.

Die Herzklappenprothese ist vorzugsweise als Taschenklappe mit drei Klappensegeln, besonders bevorzugt mit Klappensegeln aus biologischem Material, ausgebildet, wobei sich die Bögen des Nahtringes im Wesentlichen parallel zu dem dem zweiten Axialende zugewandten Befestigungsrand der Klappensegel erstrecken. Die Klappensegel sind üblicherweise an bogenförmigen Abschnitten eines Klappengestells befestigt und die Bögen des Nahtringes erstrecken sich parallel zu den Bögen dieses Klappengestells.

Die Übergangsbereiche des Nahtringes, in welchem die Laschen angeordnet sind, liegen vorzugsweise an den Umfangspositionen der Herzklappenprothese, an welchen die Kommissuren der Herzklappenprothese gelegen sind. So ist eine optimale Anpassung an die natürliche Anatomie möglich.

Die Laschen weisen vorzugsweise jeweils eine im Wesentlichen dreieckige Form auf und sind entlang einer in Umfangsrichtung der Herzklappenprothese verlaufenden Seitenkante zu dem zweiten Axialende hin umklappbar. Diese Seitenkante, um welche die Lasche umklappbar ist, ist vorzugsweise die längste Seitenkante der Lasche. Die Seitenkante verläuft dabei in Umfangsrichtung gekrümmt.

Nachfolgend wird die Erfindung beispielhaft anhand der beigefügten Figuren beschrieben. In diesen zeigt:
- Fig. 1: eine schematische perspektivische Ansicht einer erfindungsgemäßen Herzklappenprothese mit dem Nahtring in einem ersten Zustand,
- Fig. 2: eine schematische perspektivische Ansicht einer erfindungsgemäßen Herzklappenprothese mit dem Nahtring in einem zweiten Zustand und
- Fig. 3: eine gegenüber Fig. 2 um 90° gedrehte Seitenansicht der Herzklappenprothese in dem in Fig. 2 gezeigtem Zustand.

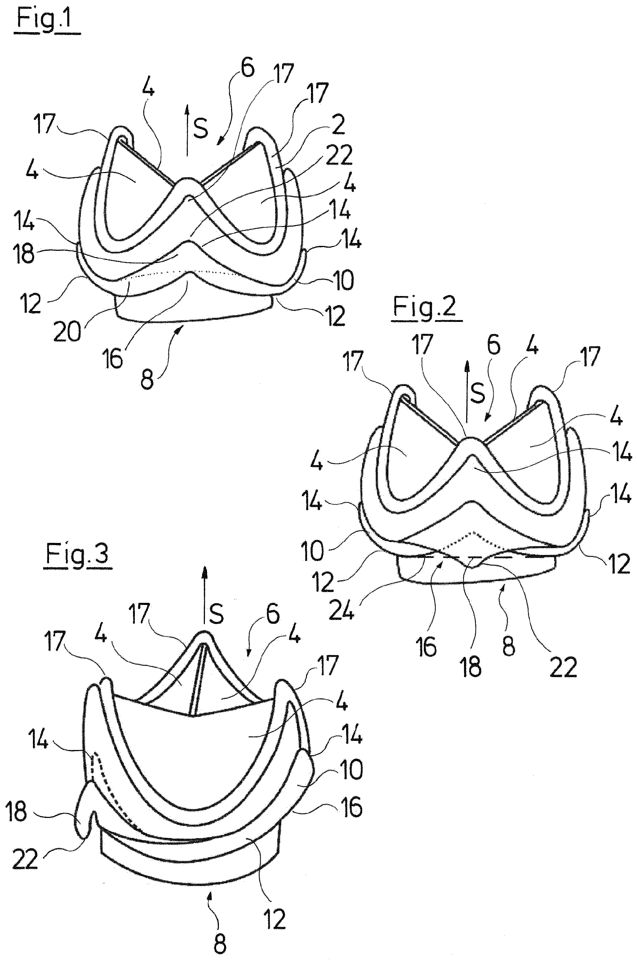

Die erfindungsgemäße Herzklappenprothese, welche in Fig. 1 gezeigt ist, weist ein Klappengestell 2 auf, an welchem drei Klappensegel 4 aus biologischem Material befestigt sind. An dem in Strömungsrichtung S stromabwärtigen ersten Axialende 6 stoßen die Klappensegel 4 in bekannter Weise aneinander. An dem entgegengesetzten stromaufwärtigen zweiten Axialende 8 ist ein Befestigungsbereich ausgebildet, in welchem ein Nahtring 10 gelegen ist. Der Nahtring 10 ist in bekannter Weise aus einem durchstechbaren Material, insbesondere einen Gewebematerial, ausgebildet und dient dazu, die Herzklappenprothese mit dem Gewebe eines Blutgefäßes zu vernähen. Der gezeigte Nahtring 10 ist in dem in Fig. 1 gezeigten Ausgangszustand bogenförmig ausgebildet, wobei er von drei Bögen 12 gebildet wird, von welchen immer zwei Bögen 12 jeweils in einem Übergangsbereich 14 aneinanderstoßen bzw. ineinander übergehen. Der Übergangsbereich 14 weist eine zum ersten Axialende 6 hin gerichtete Spitze auf und ist zum zweiten Axialende 8 hin V-förmig geöffnet, so dass zwischen den benachbarten Bögen 12 dem zweiten Axialende 8 zugewandt ein Freiraum 16 bzw. freier Zwickel 16 verbleibt. Der Freiraum 16 bildet somit eine vom zweiten Axialende 8 ausgehende Einbuchtung in den Nahtring 10. Der so geschaffene bogenförmige Verlauf des Nahtringes ist so ausgestaltet, dass die Übergangsbereiche 14 im Bereich der Kommissuren 17 der Herzklappenprothese liegen und die Bögen 12 sich parallel zu den Bögen des Klappengestells 2, an welchen die Klappensegel 4 befestigt sind, erstrecken. So wird ein Verlauf des Nahtringes 10 geschaffen, welcher im Wesentlichen dem natürlichen Verlauf des Randes der Klappensegel einer normalen Aortenklappe entspricht. Zum Ersatz einer normal ausgebildeten Aortenklappe kann der Nahtring in dieser Form verwendet werden, da so die Herzklappenprothese mit dem Rand der natürlichen Klappensegel vernäht werden kann.

In dem in Fig. 1 gezeigten Zustand weist der Nahtring 10 in seinem Verlauf eine im Wesentlichen konstante Breite auf, wobei insbesondere auch die dem zweiten Axialende 8 zugewandte Seitenkante des Nahtringes 10 den beschriebenen bogenförmigen Verlauf aufweist. In diesem Zustand bilden die freien Enden 22 der Laschen 18 die dem ersten Axialende 6 zugewandten Spitzen bzw. Enden des Nahtringes 10.

Um die Aortenklappen-Prothese, welche hier gezeigt ist, jedoch auch bei abweichenden, insbesondere asymmetrischen Anatomien, verwenden zu können, weist der Nahtring 10 in den Übergangsbereichen 14 Laschen 18 auf, welche frei beweglich sind. In diesem Ausführungsbeispiel ist nur in einem Übergangsbereich 14 eine Lasche 18 gezeigt, es ist jedoch zu verstehen, dass die zwei anderen Übergangsbereiche 14 in gleicher Weise ausgebildet sein können. Die Lasche 18 ist im Wesentlichen dreieckig und entlang ihrer längsten, sich in Umfangsrichtung erstreckenden Seitenkante 20 mit dem Nahtring 10 verbunden. Die Lasche 18 ist derart frei beweglich, dass sie in zwei Positionen geklappt werden kann, wobei eine erste Position in Fig. 1 und die zweite Position in Fig. 2 und 3 gezeigt ist. In der in Fig. 1 gezeigten Position ist die Lasche 18 mit ihrer Spitze, das heißt ihrem freien Ende 22, zu dem ersten Axialende 6 hin geklappt. In dieser Position liegt die Lasche 18 vollständig in dem bogenförmigen Verlauf des Nahtringes 10, so dass ein entsprechend bogenförmiger Nahtbereich geschaffen wird und der Freiraum 16 als Einbuchtung frei bleibt.

In der in Fig. 2 und 3 gezeigten zweiten Lage ist die Lasche 18 mit ihrem freien Ende 22 zu dem zweiten Axialende 8 hin umgeklappt, wobei die Lasche 18 um ihre lange in Umfangsrichtung verlaufende Seitenkante 20 geklappt ist. So ragt die Lachse 18 mit ihrem freien Ende in den Freiraum 16 hinein und überdeckt diesen. Das freie Ende 22 der Lasche 18 erstreckt sich dabei über eine Umfangslinie 24 hinaus welche durch oder über die Scheitelpunkte der Bögen 12 verläuft. Das heißt das freie Ende 22 erstreckt sich in der axialen Richtung zu dem zweiten Axialende 8 genau so weit oder weiter als die Bögen 12. Da so der Freiraum 16 zumindest zu einem großen Teil überdeckt wird, ist es möglich, einen ringförmigen bzw. kreisbogenförmigen Nahtverlauf, welcher parallel zu der Umfangslinie 24 verläuft, zu wählen. Das heißt die Herzklappenprothese muss nicht entlang dem sonst üblichen bogenförmigen Verlauf in dem Übergangsbereich 14 vernäht werden. So ist ein einfaches Einnähen auch bei asymmetrischen Anatomien möglich, ohne das Blutgefäß oder die Herzklappenprothese verformen zu müssen.

### Bezugszeichenliste

- 2: - Klappengestell
- 4: - Klappensegel
- 6: - erstes Axialende
- 8: - zweites Axialende
- 10: - Nahtring
- 12: - Bögen
- 14: - Übergangsbereiche
- 16: - Freiraum
- 17: - Kommissuren
- 18: - Lasche
- 20: - Seitenkante
- 22: - freies Ende
- 14: - Umfangslinie
- S: - Strömungsrichtung

## Patentansprüche

1. Herzklappenprothese mit einem stromabwärtigen ersten Axialende (6) und einem stromaufwärtigen zweiten Axialende (8) und einem bogenförmig verlaufenden Nahtring (10) zum Vernähen in einem Blutgefäß, dessen bogenförmiger Verlauf von drei Bögen (12) und drei Übergangsbereichen (14) gebildet wird, an welchen jeweils zwei der Bögen (12) mit ihren Enden ineinander übergehen, **dadurch gekennzeichnet, dass** an zumindest einem dieser drei Übergangsbereiche (14) der Nahtring (10) eine bewegliche Lasche (18) aufweist, welche eine größere freie Länge als die übrigen Bereiche des Nahtringes hat und welche wahlweise zu dem ersten Axialende (6) gerichtet oder zu dem zweiten Axialende (8) hin umgeklappt werden kann.

2. Herzklappenprothese nach Anspruch 1, **dadurch gekennzeichnet, dass** an jedem der drei Übergangsbereiche (14) eine bewegliche Lasche (18) angeordnet ist, welche wahlweise zu dem ersten Axialende (6) gerichtet oder zu dem zweiten Axialende (8) hin umgeklappt werden kann.

3. Herzklappenprothese nach Anspruch 1 oder 2, **dadurch gekennzeichnet, dass** der Nahtring, wenn die Lasche (18) oder die Laschen (18) zu dem ersten Axialende (6) hin gerichtet sind, streifenförmig ausgebildet ist und entlang seinem bogenförmigen Verlauf eine im Wesentlichen konstante Breite aufweist.

4. Herzklappenprothese nach einem der vorangehenden Ansprüche, **dadurch gekennzeichnet, dass** der Nahtring (10) an seiner dem zweiten Axialende (8) zugewandten Seite eine bogenförmige aus drei Bögen (12) gebildete Kontur aufweist.

5. Herzklappenprothese nach Anspruch 4, **dadurch gekennzeichnet, dass** die Laschen (18) jeweils so ausgebildet sind, dass sie, wenn sie zu dem zweiten Axialende (8) hin umgeklappt sind, eine zwischen zwei aneinander angrenzenden Bögen (12) gebildete Einbuchtung (16) des Nahtringes (10) zumindest teilweise überdecken.

6. Herzklappenprothese nach einem der vorangehenden Ansprüche, bei welcher die Laschen (18) derart geformt sind, dass sie, wenn sie zu dem zweiten Axialende (8) hin umgeklappt sind, sich in axialer Richtung zu diesem zweiten Axialende (8) hin zumindest bis zu einer Umfangslinie (24) erstrecken, welche durch die dem zweiten Axialende (8) zugewandten Scheitelpunkte der Bögen (12) des Nahtringes (10) verläuft.

7. Herzklappenprothese nach einem der vorangehenden Ansprüche, **dadurch gekennzeichnet, dass** der Nahtring (10), wenn die Laschen (18) zu dem zweiten Axialende (8) hin umgeklappt sind, einen im Wesentlichen kreisringförmigen Nahtbereich definiert, entlang welchem die Herzklappenprothese mit einem Blutgefäß vernäht werden kann.

8. Herzklappenprothese nach einem der vorangehenden Ansprüche, **dadurch gekennzeichnet, dass** der Nahtring (10), wenn die Laschen (18) zu dem ersten Axialende (6) hin gerichtet sind, einen bogenförmigen Nahtbereich definiert, welcher derart verläuft, dass er ein Vernähen in der Aorta entlang dem Verlauf des Randes der natürlichen Klappensegel der Aortenklappe ermöglicht.

9. Herzklappenprothese nach einem der vorangehenden Ansprüche, **dadurch gekennzeichnet, dass** die Laschen (18), wenn sie zu dem ersten Axialende (6) hin gerichtet sind, die dem ersten Axialende (6) zugewandten axialen Endbereiche des Nahtringes (10) definieren.

10. Herzklappenprothese nach einem der vorangehenden Ansprüche, **dadurch gekennzeichnet, dass** sie als Taschenklappe mit drei Klappensegeln (4), vorzugsweise aus biologischem Material, ausgebildet ist, wobei sich die Bögen (12) des Nahtringes (10) im Wesentlichen parallel zu dem dem zweiten Axialende (8) zugewandten Befestigungsrand der Klappensegel (4) erstrecken.

11. Herzklappenprothese nach einem der vorangehenden Ansprüche, **dadurch gekennzeichnet, dass** die Übergangsbereiche (14) des Nahtringes (10) mit Laschen (18) an den Umfangspositionen der Kommissuren (17) der Herzklappenprothese gelegen sind.

12. Herzklappenprothese nach einem der vorangehenden Ansprüche, **dadurch gekennzeichnet, dass** die Laschen (18) jeweils eine im Wesentlichen dreieckige Form aufweisen und entlang einer in Umfangsrichtung der Herzklappenprothese verlaufenden Seitenkante (20) zu dem zweiten Axialende (8) hin umklappbar sind.

## Claims

1. A heart valve prosthesis with a downstream first axial end (6) and with an upstream second axial end (8) and with a sewing ring (10) which runs in an arched manner for sewing in a blood vessel, the arched course of said sewing ring being formed by three arches (12) and three transition regions (14) at which two of the arches (12) with their ends merge into one another, **characterised in that** the sewing ring (10) comprises a movable tab (18) on at least one of these three transition regions (14), said tab having a greater free length that the other regions of the sewing ring and being able to be selectively directed to the first axial end (6) or folded over towards the second axial end (8).

2. A heart valve prosthesis according to claim 1, **characterised in that** a movable tab (18) which can be selectively directed to the first axial end (6) or folded over towards the second axial end (8) is arranged on each of the three transition regions (14).

3. A heart valve prosthesis according to claim 1 or 2, **characterised in that** the sewing ring is formed in a strip-like manner and along its arched course has an essentially constant width, when the tab (18) or tabs (18) are directed to the first axial end (6).

4. A heart valve prosthesis according to one of the preceding claims, **characterised in that** the sewing ring (10) at its side which faces the second axial end (8) has an arched contour which is formed from three arches (12).

5. A heart valve prosthesis according to claim 4, **characterised in that** the tabs (18) are each formed such that when they are folded over towards the second axial end (8), they at least partly cover an indentation (16) of the sewing ring (10) which is formed between two arches (12) which are adjacent to one another.

6. A heart valve prosthesis according to one of the preceding claims, **characterised in that** the tabs (18) are shaped in a manner such that when they are folded over towards the second axial end (8) they extend in the axial direction towards this second axial end (8) at least up to a peripheral line (24) which runs through the apex points of the arches (12) of the sewing ring (10), said apex points facing the second axial end (8).

7. A heart valve prosthesis according to one of the preceding claims, **characterised in that** when the tabs (18) are folded over towards the second axial end (8), the sewing ring (10) defines an essentially annulus-shaped sewing region, along which the heart valve prosthesis can be sewn to a blood vessel.

8. A heart valve prosthesis according to one of the preceding claims, **characterised in that** when the tabs (18) are directed to the first axial end (6), the sewing ring (10) defines an arched sewing region which runs in a manner such that it permits a sewing in the aorta along the course of the edge of a natural valve leaflet of the aortic valve.

9. A heart valve prosthesis according to one of the preceding claims, **characterised in that** when they are directed to the first axial end (6), the tabs (18) define the axial end regions of the sewing ring (10) which face the first axial end (6).

10. A heart valve prosthesis according to one of the preceding claims, **characterised in that** it is designed as a semilunar valve with three valve leaflets (4), preferably of biological material, wherein the arches (12) of the sewing ring (10) extend essentially parallel to the fastening edge of the valve leaflets (4) which face the second axial end (8).

11. A heart valve prosthesis according to one of the preceding claims, **characterised in that** the transition regions (14) of the sewing ring (10) with the tabs (18) are situated at the peripheral positions of the commissures (17) of the heart valve prosthesis.

12. A heart valve prosthesis according to one of the preceding claims, **characterised in that** the tabs (18) each have an essentially triangular shape and can be folded over along a side edge (20) which runs in the peripheral direction of the heart valve prosthesis, towards the second axial end (8).

## Revendications

1. Prothèse valvulaire cardiaque présentant une première extrémité axiale (6) située en aval et une seconde extrémité axiale (8) située en amont, ainsi qu'un anneau de suture (10) en forme d'arc utilisé pour effectuer une suture dans un vaisseau sanguin, dont le profil en arc est formé de trois arcs (12) et de trois zones de transition (14) au niveau desquelles respectivement deux des arcs (12) se fondent l'un dans l'autre par leurs extrémités, **caractérisée en ce que** l'anneau de suture (10) présente, au niveau d'au moins l'une de ces trois zones de transition (14), une languette (18) mobile ayant une longueur libre plus grande que les autres zones de l'anneau de suture et qui peut, au choix, être dirigée vers la première extrémité axiale (6) ou être rabattue vers la seconde extrémité axiale (8).

2. Prothèse valvulaire cardiaque selon la revendication 1, **caractérisée en ce qu'**est disposée, au niveau de chacune des trois zones de transition (14), une languette mobile (18) qui peut, au choix, être dirigée vers la première extrémité axiale (6) ou être rabattue vers la seconde extrémité axiale (8).

3. Prothèse valvulaire cardiaque selon la revendication 1 ou 2, **caractérisée en ce que** l'anneau de suture, lorsque la languette 18 ou les languettes (18) sont dirigées vers la première extrémité axiale (6), est réalisé en forme de bande et présente, le long de son profil en arc, une largeur sensiblement constante.

4. Prothèse valvulaire cardiaque selon l'une des revendications précédentes, **caractérisée en ce que** l'anneau de suture (10) présente, sur sa face orientée vers la seconde extrémité axiale (8), un contour en arc formé de trois arcs (12).

5. Prothèse valvulaire cardiaque selon la revendication 4 **caractérisée en ce que** les languettes (18) sont respectivement configurées de telle sorte que, lorsqu'elles sont rabattues vers la seconde extrémité axiale (8), elles recouvrent au moins partiellement un creux (16) de l'anneau du suture (10) formé entre deux arcs (12) adjacents.

6. Prothèse valvulaire cardiaque selon l'une des revendications précédentes, dans laquelle les languettes (18) sont conformées de telle sorte que, lorsqu'elles sont rabattues vers la seconde extrémité axiale (8), elles s'étendent en direction axiale vers cette seconde extrémité axiale (8) au moins jusqu'à une ligne périphérique (24) qui passe par les sommets, orientés vers la seconde extrémité axiale (8), des arcs (12) de l'anneau de suture (10).

7. Prothèse valvulaire cardiaque selon l'une des revendications précédentes, **caractérisée en ce que** l'anneau de suture (10), lorsque les languettes (18) sont rabattues vers la seconde extrémité axiale (8), définit une région de suture de forme sensiblement annulaire, le long de laquelle la prothèse valvulaire cardiaque peut être suturée à un vaisseau sanguin.

8. Prothèse valvulaire cardiaque selon l'une des revendications précédentes, **caractérisée en ce que** l'anneau de suture (10), lorsque les languettes (18) sont dirigées vers la première extrémité axiale (6), définit une région de suture en forme d'arc qui s'étend de façon à permettre une suture dans l'aorte le long du bord du feuillet naturel de la valve aortique.

9. Prothèse valvulaire cardiaque selon l'une des revendications précédentes, **caractérisée en ce que** les languettes (18), lorsqu'elles sont dirigées vers la première extrémité axiale (6), définissent les régions terminales axiales de l'anneau de suture (10) orientées vers la première extrémité axiale (6).

10. Prothèse valvulaire cardiaque selon l'une des revendications précédentes, **caractérisée en ce qu'**elle est réalisée en tant que valve semi-lunaire dotée de trois feuillets (4), de préférence en matériau biologique, les arcs (12) de l'anneau de suture (10) s'étendant de manière sensiblement parallèle au bord de fixation du feuillet (4) orienté vers la seconde extrémité axiale (8).

11. Prothèse valvulaire cardiaque selon l'une des revendications précédentes, **caractérisée en ce que** les zones de transition (14) de l'anneau de suture (10) pourvu de languettes (18) sont situées au niveau des positions périphériques des commissures (17) de la prothèse valvulaire cardiaque.

12. Prothèse valvulaire cardiaque selon l'une des revendications précédentes, **caractérisée en ce que** les languettes (18) présentent sensiblement une forme de triangle et peuvent être rabattues vers la seconde extrémité axiale (8) le long d'une arête latérale (20) qui s'étend dans la direction périphérique de la prothèse valvulaire cardiaque.
